(19) Europäisches Patentamt
European Patent Office
Office européen des brevets

(11) Publication number: 0 087 021 B1

(12) **EUROPEAN PATENT SPECIFICATION**

(45) Date of publication of patent specification: **10.06.87**

(51) Int. Cl.[4]: **C 12 P 17/06,** C 07 F 9/113 // A61K31/665 ,(C12P17/06, C12R1:465)

(21) Application number: **83100981.6**

(22) Date of filing: **02.02.83**

(54) **CL 1565 Antibiotic compounds and their production.**

(30) Priority: **24.02.82 US 351704**
**08.11.82 US 439973**
**07.12.82 US 447544**

(43) Date of publication of application:
**31.08.83 Bulletin 83/35**

(45) Publication of the grant of the patent:
**10.06.87 Bulletin 87/24**

(84) Designated Contracting States:
**AT BE CH DE FR GB IT LI LU NL SE**

(56) References cited:
**US-A-4 237 053**

(73) Proprietor: **WARNER-LAMBERT COMPANY**
**201 Tabor Road**
**Morris Plains New Jersey 07950 (US)**

(72) Inventor: **Stampwala, Suresh S.**
**2115 Hopkins Dr.**
**Sterling Heights, Mi. 48077 (US)**
Inventor: **French, James C.**
**3150 Rumsey**
**Ann Arbor, Mi. 48105 (US)**
Inventor: **Tunac, Josefino B.**
**4637 Fairmont**
**Troy, Mi. 48096 (US)**
Inventor: **Hurley, Timothy R.**
**3735 Greenbrier Dr. Apt. 220-B**
**Ann Arbor, Mi. 48105 (US)**
Inventor: **Bunge, Richard H.**
**2520 Traver Rd.**
**Ann Arbor, Mi. 48105 (US)**

(74) Representative: **Patentanwälte Schaumburg & Thoenes**
**Mauerkircherstrasse 31 Postfach 86 07 48**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 087 021 B1

**Description**

The present invention relates to novel, phosphorus containing antibiotics that are antitumor agents, designated CL 1565—A and CL 1565—B, and CL 1565—T, to pharmaceutically acceptable salts thereof, and to a process for the production of said compounds. The process, more particularly, relates to a fermentation process using a strain of *Streptomyces sp.*, isolate ATCC 31906, for the production of the said CL 1565 complex of this invention. The structures of the CL 1565 antibiotic components, namely CL 1565—A, CL 1565—B, and CL 1565—T, are shown in formulas 1, 2a, and 2b, respectively.

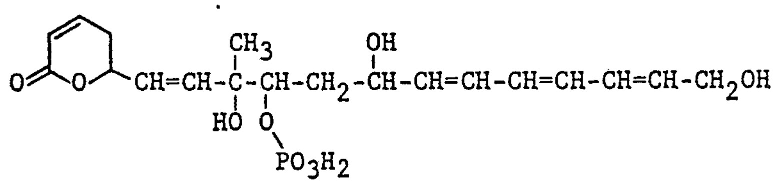

1, CL 1565-A

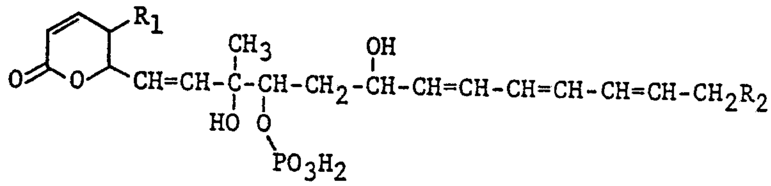

2a, $R_1$ = $R_2$ = H; CL 1565-B
2b, $R_1$ = $R_2$ = OH; CL 1565-T

The structural formulas given in 1, 2a, and 2b for CL 1565—A, CL 1565—B, and CL 1565—T, respectively, were reached by comparison of the physical and spectral characteristics of CL 1565—A, B, and T and some of their derivatives. In addition, the invention relates to pharmaceutical compositions containing various salts of the compounds of the invention alone or in combination with other antitumor agents in the treatment of neoplastic diseases.

Culture characterization and fermentation processes

In accordance with the present invention, CL 1565 compounds are produced by cultivating a selected CL 1565-complex producing strain of a *Streptomyces sp.*, isolate ATCC 31906, under artificial conditions in a suitable nutrient medium until a substantial quantity of CL 1565 compound or compounds is formed and isolating one or more of the compounds in free acid or salt form.

The strain of *Streptomyces* suitable for the purpose of this invention was found in a soil sample collected in Sao Paulo, Brazil. This organism was isolated from the soil sample using a suitable agar plating medium. An example of such a medium is one which contains salts such as potassium phosphate, magnesium sulfate, and ferrous sulfate, and carbon substrates such as glycerol and asparagine. The soil was pretreated with calcium carbonate before it was plated on the agar medium and incubated under a favorable temperature, particularly 24°C, to allow the development of the soil microorganisms.

The CL 1565-complex pruducing organism that was isolated is an unidentified strain of *Streptomyces* and has been deposited with the American Type Culture Collection, Rockville, Maryland 20852, and is being maintained in their permanent culture collection as ATCC 31906. This organism that produces the CL 1565 compounds is maintained as a dormant culture in lyophile tubes, cryogenic vials, and in soil tubes at the Warner-Lambert/Parke-Davis Culture Laboratory and is designated as isolate WP—426.

The antitumor compound CL 1565—A, CL 1565—B and CL 1565—T, are produced by isolate WP—426 during aerobic fermentation under controlled conditions. The fermentation medium consists of sources of carbon, nitrogen minerals, and growth factors. Examples of carbon sources are various simple sugars such as cerelose, mannose, fructose, xylose, ribose, and glycerol, or other carbohydrate containing compounds such as dextrin, starch, corn meal, and whey. The normal quantity of the carbon sources varies from 0.1 to 10 percent by weight.

The nitrogen sources are organic, inorganic, or mixed organic-inorganic in nature. Examples of such compounds are cotton seed meal, soybean meal, corn germ flour, corn steep liquor, distillers solubles, peanut meal, peptonized milk, and various ammonium salts. The normal amount added varies from 0.1 to 5%, but higher amounts are also acceptable.

The inclusion of minerals and growth factors in the fermentation medium is also helpful in the production of CL 1565—A, B and T. Examples of media ingredients that provide minerals are potassium phosphate, sodium chloride, ferrous sulfate, calcium carbonate, cobalt chloride, and zinc sulfate. The sources of growth factors include various yeast and milk products.

The preferred method for producing the CL 1565 compounds is by submerged culture fermentation. According to an embodiment of this invention, fermentation ingredients are prepared in solution and sterilized by autoclaving or steam heating. The pH of the aqueous medium is preferably between 4 and 8. The fermetation medium is cooled to a suitable temperature, between 16 and 45°C, and then inoculated with the suitable culture. Fermentation is carried out with aeration and agitation, and the maximum production of CL 1656 compounds is usually reached in 2 to 10 days. Fermentation in solid state can also be used.

In the submerged culture method, fermentation is carried out in shake flask or in stationary tank fermentors. In shake flasks, aeration is brought about by agitation of the flasks which causes mixing of the medium with air. In the stationary fermentors, agitation is provided by impellers in the form of disc turbines, vaned discs, open turbines, or marine propellers. Aeration is accomplished by injecting air or oxygen into the fermentation mixture.

The examples which follow illustrate the preferred methods by which the CL 1565 products according to the invention are produced. The described processes are capable of wide variation, and any minor departure or extension is considered as within the scope of this invention.

## Example 1

Seed development and shake flask fermentation

The culture in its dormant stage is transferred to a CIM—23 agar slant and incubated for 7—14 days at 28°C. A portion of the microbial growth from the slant is used to inoculate and 18 x 150 mm seed tube containing 5 ml of ARM 1550 seed medium. The seed tube is shaken at 24°C for 3—4 days.

| <u>CIM 23 agar slant</u> | |
|---|---|
| Amidex® corn starch | 10 g |
| N—Z amine, type A | 2 g |
| Beef Extract (Difco) | 1 g |
| Yeast Extract (Difco) | 1 g |
| Cobaltous chloride · $6H_2O$ | 0.020 g |
| Agar | 20 g |
| Distilled water | 1000 ml |

| <u>ARM 1550 medium</u> | % |
|---|---|
| Bacto-Yeast Extract (Difco) | 0.5 |
| Glucose, Monohydrate | 0.1 |
| Soluble Starch (Difco) | 2.4 |
| Bacto-Tryptone (Difco) | 0.5 |
| Bacto-Beef Extract (Difco) | 0.3 |
| $CaCO_3$ | 0.2 |

NOTE: Adjust pH to 7.5 with NaOH before adding $CaCO_3$

A portion (1 ml) of the microbial growth from the seed tube is transferred to a 300 ml Erlenmeyer baffled shake flask containing 50 ml of SM 64 production medium. The inoculated flask is incubated at 24°C for 5 days with shaking using a gyratory shaker (2" [5,08 cm] throw) set at 180 RPM. The fermentation beer after five days of fermentation is tan in color, the mycelia are granular in appearance, and the pH of the fermentation beer is about 5.5.

SM 64 Production Medium

| | |
|---|---|
| Whey (Kroger Dairy) | 35.0% by volume |
| Dextrin (Amidex® B411), American Maize | 1.5% by weight |
| Pharmadia® (traders Protein) 431307 | 1.5% by weight |
| Distilled water | |

NOTE: Adjust pH to 6.5 with sodium hydroxide

The fermentation broth is assayed at a 1:100 dilution vs L1210 mouse leukemia cells *in vitro*. A 0-50% growth of cells compared with an L1210 cell control is considered to indicate activity, with 0% being the most active. The cytoxicity of two experimental shake flask fermentations were:

| | Cytoxicity (% Growth) | |
|---|---|---|
| Flask Number | Supernatant | Freeze-dried |
| I. | 28 | 14 |
| II. | 17 | 30 |

The above fermentation broths were also tested vs several microorganisms using the agar-disc method. The crude broth was found to be active vs *Neisseria catarrhalis* 03596, *Staphylococcus aureus* 02482, *Bacillus subtilis* 04555, *Kloeckera brevis* M1378, *Rhodotorula glutinis* M1384, *Saccharomyces cerevisiae* 01525, and *Pencillium avellaneum* M2988.

Example 2

Fermentation in 757 liter (200-gallon) fermentors

Seed development

A cryogenic vial containing approximately 1 ml of culture suspension is used as the source of inoculum. The contents of this cryogenic vial are thawed and aseptically transferred to a two liter, baffled Erlenmeyer flask containing 500 ml of SD-05 seed medium. The inoculated flask is incubated for 46-48 hours at 24°C, on a gyratory shaker, at 130 RPM speed.

| SD—05 Seed Medium | % |
|---|---|
| Amberex® 1003 (Amber Labs) | 0.5 |
| Glucose Monohydrate (Cerelose) | 0.1 |
| Dextrin-Amidex® B411 (Corn Products) | 2.4 |
| N—Z Case (Humko Sheffield) | 0.5 |
| Spray Dried Meat Solubles (Daylin Labs) | 0.3 |
| $CaCO_3$ | 0.2 |
| Distilled water | |

After 48 hours, the contents of the seed flask are transferred aseptically to a 30-liter, stainless steel fermentor containing 16 liters of SD—05 seed medium. The inoculated fermentor is incubated for 18—24 hours at 24°C, stirred at 300 RPM, and sparged with air at 1 VVM rate. This microbial growth is used to inoculate the 757 l (200-cal) production fermentor.

Production Fermentors

A 757 l (200-gal) fermentor which contains 605,7 l (160-gal) of SM 64 is sterilized by heating with steam for 40 min. at 121°C. The medium is cooled to 24°C and then inoculated with about 16 liters of the microbial growth from the 30-liter seed fermentor. The inoculated medium is allowed to ferment for five to seven days at 24°C, 190 RPM agitation, and sparged with 1 VVM air. Antifoam agents, Dow Corning "C" and polyglycol P—2000, are used to control foaming.

The production of CL 1565—A and at least two related antitumor antibiotics, namely CL 1565—B and CL 1565—T, are monitored throughout the fermentation cycle by recording fermentation parameters such as

pH and percent sedimentation or growth as well as by *in vitro* assays vs L1210 mouse leukemia cells and a high pressure liquid chromatographic procedure described later. An example of a fermentation profile in a 757 l (200-gal) fermentor is shown in the following table.

| Fermentation Time (hr) | pH | % Sedimentation (growth) | % Growth of L1210 Cells 1:100 | 1:300 | 1:1000 | Micrograms CL 1565—A/ml (HPLC Assay) |
|---|---|---|---|---|---|---|
| 0 | 6.0 | 0 | — | — | — | — |
| 12 | 5.8 | 3.6 | — | — | — | — |
| 24 | 5.1 | 13.3 | NA* | — | — | — |
| 36 | 5.15 | 14.7 | NA | — | — | — |
| 48 | 5.35 | 19.3 | NA | NA | NA | — |
| 72 | 5.45 | 22.0 | NA | NA | NA | 3—6 |
| 96 | 5.95 | 24.7 | 18.2 | 52.9 | NA | 10—20 |
| 118 | 7.65 | 43.3 | 0 | 30.2 | NA | 50—65 |
| 132 | 7.80 | 39.3 | 0 | 23.9 | NA | 60—65 |
| 142 | 7.90 | 40.0 | 0 | 17.2 | NA | 60—70 |

NA* = not active

This fermentor was harvested after 142 hours of fermentation with a harvest volume of 530 L (140 gal).

Isolation of CL 1565—A

Example 3

The harvested beer from the above fermentation was mixed with 34 kg of Celite 545 and filtered through a plate and frame filter press. The filtrate (473 liters) was percolated through a 30.5 cm [O.D.) column containing 120 liters of HP—20 resin (Gillies International, Inc., La Jolla, California). The resin was then washed with water (605 liters), and 90:10 water:methanol (170 liters). Most of the CL 1565—A was then eluted from the resin with 80:20 water:methanol. High pressure liquid chromatographic analyses (HPLC), performed in the manner described below, of the ensuing eluates showed the following elution profile.

| 8:20 water:methanol eluate | grams of CL 1565—A |
|---|---|
| #1 = 340 liters | <2. g |
| #2 = 340 liters | 11.5 g |
| #3 = 340 liters | 7.0 g |

Eluates #2 and #3 were separately concentrated and lyophilized to afford 90.2 g and 78.7 g, respectively, of dark brown solids. These products were combined and dissolved in 3 liters of water. The resulting solution was added to 27 liters of methanol with stirring. After standing overnight at 5°C, the mixture was filtered and the precipitate was washed with 5 liters of methanol. The filtrate and wash were combined, concentrated *in vacuo*, and lyophilized to yield 104.5 g of a solid. A portion of this product (95 grams) in 1.5 liters of water was added slowly with mixing to 17 liters of 1-propanol. After one hour the resulting mixture was filtered and the precipitate was washed with 2 liters of 1-propanol. The filtrate and wash were combined, concentrated, and lyophilized to afford 57 g of a solid which, by HPLC analysis, contained about 15 g of CL 1565—A.

This product was chromatographed, in approximately 15 g lots, on 1.2 liters of 40 μm $C_{18}$-silica gel (Analytichem International, Inc., Harbor City, California) contained in a 7.6 cm [O.D.] column. The eluent used was 0.005 M pH 4.5 ammonium acetate buffer followed by 0.005 M pH 4.5 ammonium acetate containing 5% acetonitrile. The fractions collected were assayed by HPLC. The fractions containing CL

1565—A were pooled, concentrated, and lyophilized. A portion (570 mg) of the resulting product was rechromatographed using a Prep LC/System 500 apparatus fitted with a Prep-Pak™-500/$C_{18}$ column (Waters Instruments, Inc., Milford, Massachusetts) and 0.1 M pH 6.5 phosphate buffer containing 10% acetonitrile as the eluent. The major fractions, containing approximately 375 kg of CL 1565—A, were pooled and concentrated *in vacuo*. The aqueous solution was passed through a column containing 200 ml of HP—20 resin packed in water. The resin was then washed with 1400 ml of water and CL 1565—A remaining on the column was eluted with 350 ml of 50% methanol. The eluate was concentrated in *in vacuo* and passed through a column containing 35 ml of Dowex®—50X2 ($Na^+$). The effluent (pH 5.5) and a water wash of the resin were combined and lyophilized to yield 180 mg of purified CL 1565—A, isolated as a sodium salt.

Analysis of this product shows that it contains approximately 1.3 moles of sodium per 1.0 mole of parent CL 1565—A free acid. Because the free acids (CL 1565—A, CL 1565—B, and CL 1565—T) are labile, it is preferred to isolate CL 1565 free acid compounds in the salt form such as the sodium salt from, preferably as the salts having about 1.0 to about 2.0 moloes of sodium per 1.0 mole of free acid.

Example 4

Filtered beer (719 liters), prepared in the same manner as described above, wash passed over 31 liters of Dowex®-1 x 2 (chloride form) packed in a 30.5 cm [O.D.] column. The effluent and the subsequent water wash did not contain any detectable amounts of the CL 1565 components. The entire fractionation described herein was monitored by the HPLC method described below using 0.1 M pH phosphate buffer ($Na^+$)-acetonitrile (88:12) as the solvent system. The Dowex®-1 resin was then eluted with 1M sodium chloride-methanol (1:1) and the eluate was collected in two 10-liter and six 15-liter fractions. Most of the CL 1565—A, CL 1565—B, CL 1565—T, and additional minor CL 1565 components appeared in eluates two through six. These fractions were combined and diluted with 246 liters of acetone. The resulting mixture was stored at 5°C overnight. The clear supernatant solution was removed and concentrated to 16 liters in vacuo. Lyophilization of this concentrate afforded 800 g of a solid. The product (740 g) was added to 552 g of a similar product isolated in the same manner and the combined solids were dissolved in 20 liters of water. The resulting solution (pH 6.0) was chromatographed on 50 liters of HP—20 resin contained in a 15 cm (O.D.) column. Elution of the HP—20 column with 175 liters of water removed most of the CL 1565—T and all of the minor, more polar CL 1565 components. Most of the CL 1565—A component was eluted with 100 liters of methanol-water (15:85); CL 1565—B and the remaining amount of CL 1565—A were eluted with 83 liters of methanol-water (50:50). The eluates richest in CL 1565—A were combined, concentrated, and lyophilized to afford 123 g of a solid which, by HPLC analysis, contained about 110 g of CL 1565—A.

A 75-gram portion of this product was dissolved in two liters of 0.05 M pH 6.8 phosphate buffer and further purified by chromatography on 52 liters (25 kg) of 100 μm $C_{18}$ reverse phase silica gel (Analytichem International, Inc. Harbor City, California) packed in 0.05 M pH 6.8 phosphate buffer ($Na^+$) in a 15 cm [O.D.] column. The column was developed with 0.05 M phosphate buffer containing increasing amounts (4.0-6.5% of acetonitrile. The early fractions contained primarily CL 1565—T and additional minor, more polar CL 1565 components. The majority of the CL 1565—A component was eluted in subsequent fractions. The fractions containing CL 1565—A as the only UV-absorbing component were pooled and concentrated in vacuo to 20 liters. This concentrate was stored overnight at 5°C and the inorganic salt that precipitated was filtered off. The filtrate was then charged on a 15 cm [O.D.] column containing 28 liters of HP—20 resin. The resin was washed with water (66 liters) and CL 1565—A was then eluted with 42 liters of methanol-water (50:50). The eluates that contained the majority of the CL 1565—A were combined (26 liters), concentrated, and lyophilized to yield 34 g of CL 1565—A containing some inorganic impurities. The inorganic impurities can be removed by dissolving the product in methanol (at 50 to 100 mg/ml), filtering off any insoluble material, and converting the filtrate to an aqueous solution by continually adding water to the filtrate as it is being concentrated in vacuo. Final purification of CL 1565—A is effected by chromatography of the resulting aqueous concentrate on HP—20 resin.

Isolation of additional CL 1565 components

Careful chromatography of the concentrates obtained form CL 1565 beers on $C_{18}$-silica gel or HP—20 resin affords fractions that contain CL 1565 components other than CL 1565—A. More than six compounds are detectable by HPLC and are related to CL 1565—A on the basis of similar ultraviolet absorption spectra. Two of these components, namely CL 1565—B and CL 1565—T were isolated as essentially pure compounds. CL 1565 components A, B, and T can be readily distinguished by HPLC on a μBondapak™ $C_{18}$-silica gel column (3.9 mm I.D. x 30 cm) using 0.05M—0.10M phosphate buffers containing varying proportions of acetonitrile at a flowrate of 1.5 ml/min and detection by ultraviolet absorption at 254 nm. Typical retention times of CL 1565—A, B, and T using the above HPLC conditions are given in the following table.

| | Retention time (min) in: | |
|---|---|---|
| | Solvent A* | Solvent B** |
| CL 1565—T | 2.8 | <1.5 |
| CL 1565—A | 4.3 | <1.5 |
| CL1565—B | >15 | 4.2 |

*0.05M pH 7.4 phosphate buffer-acetonitrile (87:13)
**0.05M pH 7.4 phosphate buffer-acetonitrile (78:22)

Crude beers can be assayed in the above manner except the solvent used is 0.1 M pH 6.8 phosphate buffer-acetonitrile (88:12). In this case, at a flowrate of 2 ml/min, the retention times of CL 1565—T, CL 1565—A, and CL 1565—B are approximately 2.7, 5.0, and > 12 minutes, respectively.

CL 1565—T is eluted earlier than CL 1565—A from HP—20 resin and from reverse phase silica gel. It can be isolated from the early fractions of the $C_{18}$-silica gel column described in example 4, above, using HP—20 resin.

CL 1565—B is eluted more slowly than CL 1565—A from HP—20 resin and from reverse phase silica gel. CL 1565—B is eluted with 50% methanol during the HP—20 chromatography of the crude Dowex®-1 product desribed in example 4, above. This component can best be isolated by rechromatography on HP—20 followed by chromatography on 40 μm $C_{18}$-silica gel using essentially the same procedure described for the purification of CL 1565—A.

Referring to the drawing,

Figure 1 is a representation of the ultraviolet absorption spectrum in MeOH of CL 1565—A, Sodium Salt;

Figure 2 is a representation of the infrared absorption spectrum in KBr of CL 1565—A, Sodium Salt;

Figure 3 is a representation of the 360 MHz proton magnetic resonance spectrum in $D_2O$ of CL 1565—A, Sodium Salt; and

Figure 4 is a representation of the $^{13}C$-nuclear magnetic resonance spectrum in $D_2O$ of CL 1565-A, Sodium Salt.

Properties of CL 1565—A, Sodium Salt

Ultraviolet Absorption Spectrum in MeOH (Figure 1)

λmax 268 nm ($a_1^1$ = 805) with inflections at 259 and 278 nm

Infrared Absorbtion Spectrum in KBr (Figure 2)

Principal absorptions at: 3400, 1710, 1630, 1420, 1387, 1260, 1155, 1090, 1060, 975, 920, 820, and 775 reciprocal centimeters.

Optical Rotation

$[\alpha]_D^{23}$ + 28.2° (1.0% in 0.1 M pH phophate buffer)

Elemental Analysis

| | %C | %H | %Na | %P |
|---|---|---|---|---|
| Calcd. for $C_{19}H_{27.7}O_{10}Na_{1.3}P$: | 47.84 | 5.86 | 6.27 | 6.49 |
| Found: | 48.01 | 5.88 | 6.05 | 6.3 |

Mass Spectrum (via fast atom bombardment)

Calcd. for $[C_{19}H_{25}Na_2O_9P+H]^+$ = m/z 475
$[C_{19}H_{26}Na\ O_9P+H]^+$ = m/z 453

Found: m/z 475, 453

360 MHz Proton Magnetic Resonance Spectrum in $D_20$ (Figure 3)

Principal signals at:

(s = singlet, d =doublet, t = triplet, m = multiplet) 1.29 s (3H), 1.58 t (1H), 1.70 m (1H), 2.49-2.58 m (2H), 4.13-4.18 m (3H), 4.86 t (1H), 5.09 m (1H) 5.53 t (1H), 5.9-6.0 m (4H), 6.14 t (1H), 6032 t (1H), 6.55 t (1H), 6.75 dd (1H), and 7.09 m (1H) parts per million downfield from sodium 2,2-dimethyl-2-silapentane-5-sulfonate (DSS).

$^{13}C$-Nuclear Magnetic Resonance Spectrum in $D_2O$ (Figure 4)

Principal signals at:

| peak number | | peak number | |
|---|---|---|---|
| 1 | 168.4 | 12 | 79.5 |
| 2 | 149.8 | 13 | 79.0 |
| 3 | 138.1 | 14 | 75.6 |
| 4 | 135.0 | 15 | 64.4 |
| 5 | 134.4 | 16 | 62.7 |
| 6 | 13.13 | 17 | 39.4 |
| 7 | 127.4 | 18 | 29.7 |
| 8 | 126.7 | 19 | 23.5 parts per million |
| 9 | 124.9 | | downfield from |
| 10 | 124.8 | | tetramethylsilane |
| 11 | 120.1 | | (TMS). |

The $^{31}P$-Nuclear Magnetic Resonance Spectrum in $D_2O$ exhibits a doublet ($J = 10$ Hz) at 0.504 ppm downfield from 85% phosphoric acid.

High Pressure Liquid Chromatography

Column: µBondapak™ $C_{18}$ silica gel (3.9 mm I.D. x 30 cm)
Solvent: 0.005M pH 7.3 sodium phosphate buffer-acetonitrile (90:10)
Flowrate: 2 ml/min
Detection: ultraviolet absorption at 254 nm
Retention time: 2.8 min

Antifungal Activity

Paper disks (12.7 mm in diameter) impregnated with an aqueous solution containing 500 µg of CL 1565—A/ml were placed on a layer of agar inoculated with the indicated microorganisms. After incubation overnight at 28°C, the following zones of inhibition were observed.

| Organism | Zone diameter |
|---|---|
| *Saccharomyces cerevisiae* | 15 mm |
| *Saccharomyces italicus* | 17 mm |
| *Saccharomycoides ludwigii* | 25 mm |

In Vitro Activity of CL 1565—A Against L1210 Leukemia Cells

$ID_{50} = 0.078$ µg/ml

In Vivo Antitumor Activity of CL 1565-A Against P388 Lymphatic Leukemia in Mice

| Dose | T/C* × 100 | | |
|---|---|---|---|
| (mg/kg/day) | test — 1 | test — 2 | test — 3 |
| 25 | — | — | 174 |
| 17 | 256 | 248 | — |
| 12.5 | — | — | 150 |
| 8.5 | 256 | 209 | — |
| 6.3 | — | — | 142 |
| 4.3 | 170 | 172 | |
| 2.1 | 173 | 157 | |
| 1.1 | 170 | 139 | |

$$*T/C = \frac{\text{median survival time of treated mice}}{\text{median survival time of control mice}}$$

The test method used is based on that described in *Cancer Chemother. Reports* 3:1—87 (part 3), 1972.

Properties of CL 1565-T, Sodium Salt

Ultraviolet Absorption Spectrum in MeOH

Nearly identical to Figure 1 with $a_1^1$ = 774 at λmax 268 nm and inflections at 260 and 278 nm.

Infrared Absorption Spectrum in KBr

Principal absorptions at: 3400, 1715, 1630, 1380, 1260, 1090, 970, 830 and 770 reciprocal centimeters.

Mass Spectrum (via fast atom bombardment)

Calcd. for $[C_{19}H_{25}Na_2O_{10}P+H]^+$ = m/z 491

Found: m/z 491

360 MHz Proton Magnetic Resonance Spectrum in $D_2O$. The $^1$H-NMR spectrum of CL 1565-T is very similar to the $^1$H-NMR spectrum of CL 1565-A with the exception that the former spectrum exhibits a characteristic one proton signal appearing as a doublet of doublets at 4.34 ppm and is devoid of any signals between 2.2—3.2 ppm downfield from DSS.

Principal Signals of CL 1565-T, sodium salt are at: (s = singlet, d = doublet, t = triplet, m = multiplet) 1.30 s (3H), 1.55—1.64 m (1H), 1.73 t (1H), 4.13—4.20 m (1H), 4.16 d (2H), 4.34 dd (1H), 4.94 t (1H), 5.09 dd (1H), 5.55 t (1H), 5.89—6.06 m (3H), 6.16 m (2H), 6.36 t (1H), 6.56 t (1H), 6.76 dd (1H), 7.14 dd (1H) parts per million downfield from DSS.

90.4 MHz $^{13}$C-Nuclear Magnetic Resonance Spectrum in $D_2O$:

| Peak Number | Chemical Shift* | Peak Number | Chemical Shift* |
|---|---|---|---|
| 1 | 24.10 | 11 | 126.91 |
| 2 | 41.60 | 12 | 127.18 |
| 3 | 64.68 | 13 | 128.99 |
| 4 | 64.90 | 14 | 133.36 |
| 5 | 66.67 | 15 | 136.87 |
| 6 | 78.28 | 16 | 137.23 |
| 7 | 79.81 | 17 | 142.27 |
| 8 | 84.33 | 18 | 149.6 |
| 9 | 124.40 | 19 | 169.66 |
| 10 | 126.21 | | |

*parts per million downfield from TMS

In Vivo Antitumor Activity of CL 1565-T Against P388 Lymphatic Leukemia in Mice

| Dose | T/C × 100 |
|---|---|
| mg/kg/day | |
| 25.0 | 157 |
| 12.5 | 147 |
| 6.3 | 142 |
| 3.1 | 147 |

Properties of CL 1565-B, Sodium Salt

Ultraviolet Absorption Spectrum in MeOH

λmax 267 nm ($a^1_1$ = 805) with inflections at 259 and 277 nm

Infrared Absorption Spectrum in KBr

Principal abnsorptions at: 1720, 1640, 1385, 1200, 1060, 970, and 820 reciprocal centimeters.

360 MHz Proton Magnetic Resonance Spectrum in $D_2O$

Principal Signals at:

(s = singlet, d = doublet, t = triplet, m = multiplet)

1.32 s (3H, 1.58 t (1H), 1.72 t (1H), 1.79 d (3H), 2.45—2.68 m (2H), 4.15 t (1H), 4.89 t (1H), 5.10 m (1H), 5.49 t (1H), 5.83—6.21 m (6H), 6.50—6.64 m (2H), 7.06—7.13 m (1H) parts per million downfield from DSS.

90.4 MHz $^{13}$C-Nuclear Magnetic Resonance Spectrum in $D_2O$:

| Peak Number | Chemical Shift* | Peak Number | Chemical Shift* |
|---|---|---|---|
| 1 | 20.70 | 11 | 127.24 |
| 2 | 25.06 | 12 | 129.47 |
| 3 | 31.91 | 13 | 129.90 |
| 4 | 41.85 | 14 | 134.66 |
| 5 | 66.85 | 15 | 135.94 |
| 6 | 77.87 | 16 | 136.67 |
| 7 | 80.87 | 17 | 140.42 |
| 8 | 81.64 | 18 | 152.01 |
| 9 | 122.41 | 19 | 170.56 |
| 10 | 124.45 | | |

*parts per million downfield from TMS

In Vivo Antitumor Activity of CL 1565—B Against P388 Lymphatic Leukemia in Mice

| Dose mg/kg/day | T/C × 100 |
|---|---|
| 25.0 | Toxic |
| 12.5 | 178 |
| 6.3 | 142 |
| 3.1 | 129 |

The antibiotic CL 1565 compounds can be used for their antimicrobial and antitumor activity in the form of pharmaceutical compositions containing any of the various metallic salts such as the sodium, potassium, calcium, or magnesium salt, and the like, or as other salts such as the ammonium salt or salts formed from suitable organic amines. Such pharmaceutical compositions are used with a compatible pharmaceutically acceptable carrier. The compositions may also contain other active antimicrobial and/or antitumor agents. The compositions may be made up in any pharmaceutical form appropriate for the route of administration in question. Examples of such compositions include solid compositions for oral administration such as tablets, capsules, pills, powders and granules, liquid compositions for topical or oral administration such as solutions, suspensions, syrups and elixirs, and preparations for parenteral administration such as sterile solutions, suspensions, or emulsions.

For use as an antimicrobial agent, the compositions are administered so that the concentration of active ingredient is greater than the minimum inhibitory concentration for the particular organism being treated.

The examples which follow illustrate preferred pharmaceutical compositions containing one or more of the products, CL 1565—A, CL 1565—B, and CL 1565—T, for treatment of diseases, especially neoplastic diseases.

Example A

For parenteral use, a sterile, lyophilized product containing in each ampoule 75 mg of CL 1565—A, sodium salt is prepared from the solution of the compound (in injectable distilled water).

Example B

| | |
|---|---|
| CL 1565—A, sodium salt | 75 mg |
| Sodium ascorbate | 33 mg |

The above components are placed in a sterile vial. An injection is prepared by dissolving the above mixture in a physiological saline solution according to customary procedures. A buffering agent could be added according to need.

Example C

CL 1565—A sodium salt (1000 mg) and sodium ascorbate (440 mg) are dissolved in 100 ml of water. The solution is filtered through a sterile filter and aseptically filled into presterilized vials and lyophilized. The vials are sealed under nitrogen with presterilized closures and stored at 5°C or lower.

Other suitable formulations can be made as described in examples A, B, and C, above, using CL 1565—B and CL 1565—T instead of CL 1565—A. For each of CL 1565—A, CL 1565—B, and CL 1565—T, a suggested dosage regimen for use as an antitumor agent in mammalian species is 1.0—100 mg per square meter for a single daily intravenous treatment course.

What we desire to claim as our exclusive property is the following.

## Claims

1. Phosphorus containing antibiotic compounds designated CL 1565—A, CL 1565—B, and CL 1565—T, having the structural formulas 1, 2a and 2b, respectively,

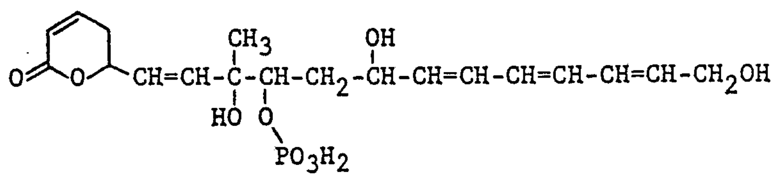

1, CL 1565-A

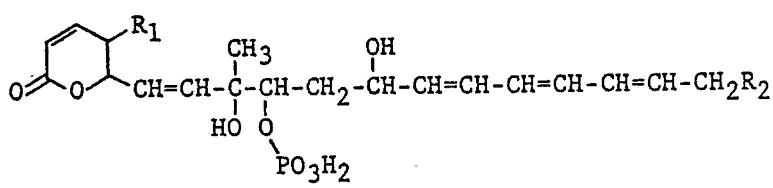

2a, $R_1 = R_2 = H$; CL 1565-B

2b, $R_1 = R_2 = OH$; CL 1565-T

and pharmaceutically acceptable salts thereof.

2. A pharmaceutically acceptable salt according to claim 1, characterized in that it is the sodium salt.

3. Process for the production of at least one CL 1565 compound according to Claim 1 comprising cultivating a CL 1565—complex producing strain of the organism *Streptomyces sp.* ATCC 31906 in a suitable medium under aerobic conditions until a substantial quantity of CL 1565 compound or compounds is formed and isolating one or more of the compounds in free acid or salt form.

4. A pharmaceutical composition comprising at least one of CL 1565—A, CL 1565—B, and CL 1565—T, and pharmaceutically acceptable salts thereof, according to Claim 1 or 2, and a pharmaceutically acceptable carrier.

## Patentansprüche

1. Phosphor enthaltende antibiotische Verbindungen mit der Bezeichnung CL 1565—A, CL 1565—B und CL 1565—T, mit den Strukturformeln 1, 2a und 2b

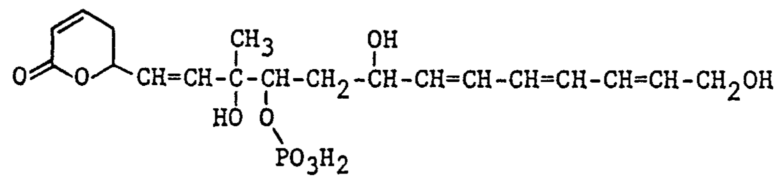

1, CL 1565-A

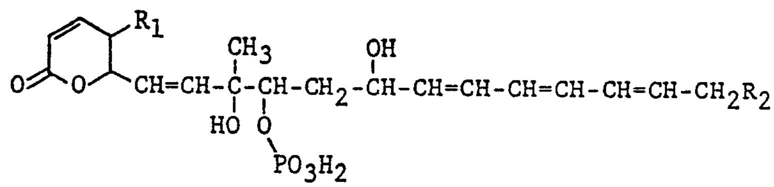

2a, $R_1 = R_2 = H$; CL 1565-B
2b, $R_1 = R_2 = OH$; CL 1565-T

und pharmazeutisch annehmbare Salze davon.

2. Pharmazeutisch annehmbares Salz gemäß Anspruch 1, dadurch gekennzeichnet, daß es das Natriumsalz ist.

3. Verfahren zur Herstellung von wenigstens einer CL 1565—Verbindung gemäß Anspruch 1, dadurch gekennzeichnet, daß man einen einen CL 1565—Komplex produzierenden Stamm des Organismus *Streptomyces sp.* ATCC 31906 in einem geeigneten Medium unter aeroben Bedingungen kultiviert, bis eine wesentliche Menge von CL 1565—Verbindung oder Verbindungen gebildet ist, und eine oder mehrere der Verbindungen in Form der freien Säure oder als Salz isoliert.

4. Pharmazeutische Zusammensetzung, enthaltend mindestens eine der Substanzen CL 1565—A, CL 1565—B und CL 1565—T, und pharmazeutisch annehmbare Salze davon, gemäß Anspruch 1 oder 2, und einen pharmazeutisch annehmbaren Träger.

**Revendications**

1. Composés antibiotiques contenant du phosphore, désignés CL 1565—A, CL 1565—B et CL 1565—T, ayant les formules structurales respectives 1, 2a et 2b

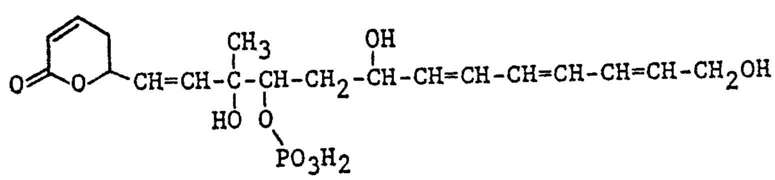

1, CL 1565-A

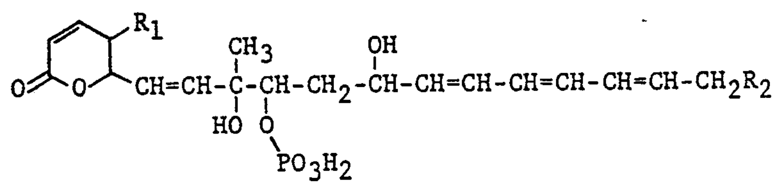

2a, $R_1 = R_2 = H$; CL 1565-B
2b, $R_1 = R_2 = OH$; CL 1565-T

et leurs sels pharmaceutiquement acceptables.

2. Sel pharmaceutiquement acceptable selon la revendication 1, caractérisé en ce que c'est le sel sodique.

3. Procédé pour préparer au moins un composé CL 1565 selon la revendication 1, consistant à cultiver une souche, produisant un complexe CL 1565, de l'organisme *Streptomyces sp.* ATCC 31 906 dans un milieu approprié et dans des conditions aérobies jusqu'à la formation d'une quantité notable d'un ou de plusieurs composés CL 1565, et à isoler un ou plusieurs de ces composés sous la forme d'un acide libre ou d'un sel.

4. Composition pharmaceutiquement contenant au moins l'un des composés CL 1565—A, CL 1565—B et CL 1565—T, ou l'un de leurs sels pharmaceutiquement acceptables, selon la revendication 1 ou 2, et un véhicule pharmaceutiqement acceptable.

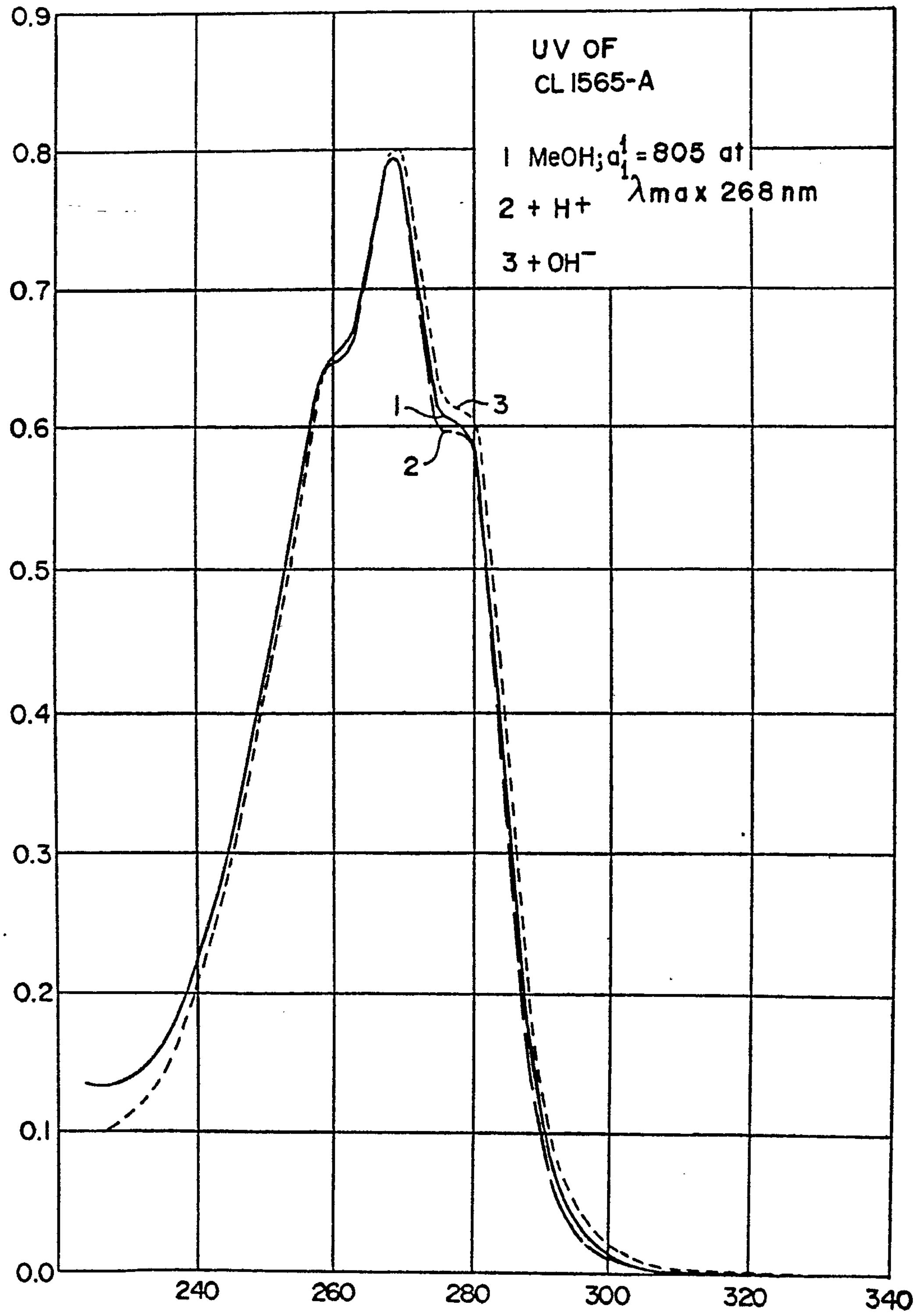
UV OF
CL 1565-A
1 MeOH; $a_1^1$ = 805 at
λmax 268 nm
2 + H+
3 + OH⁻
1
2
3
0.9
0.8
0.7
0.6
0.5
0.4
0.3
0.2
0.1
0.0
240
260
280
300
320
340

FIG. 1

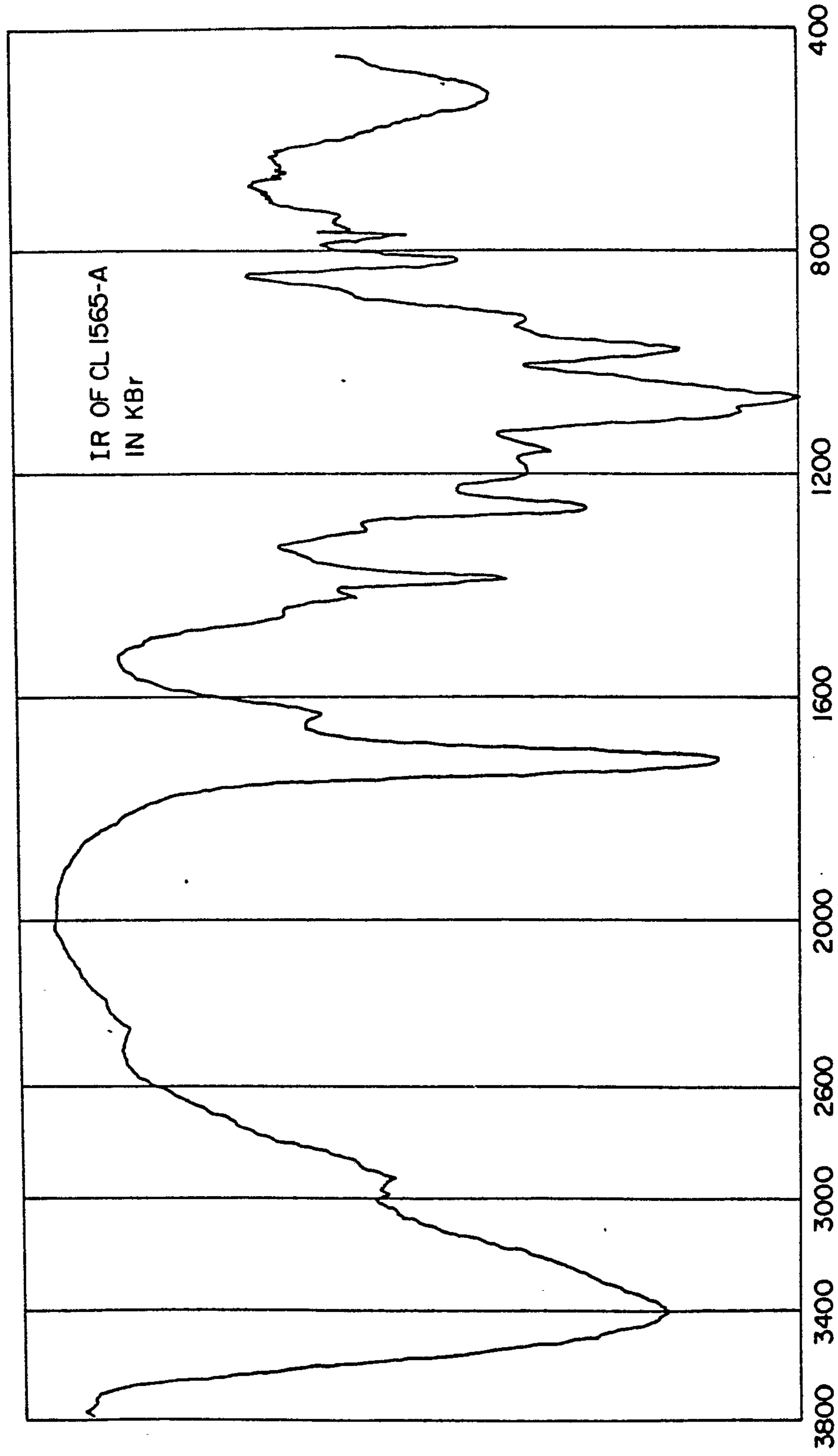
IR OF CL 1565-A
IN KBr
400
800
1200
1600
2000
2600
3000
3400
3800

FIG. 2

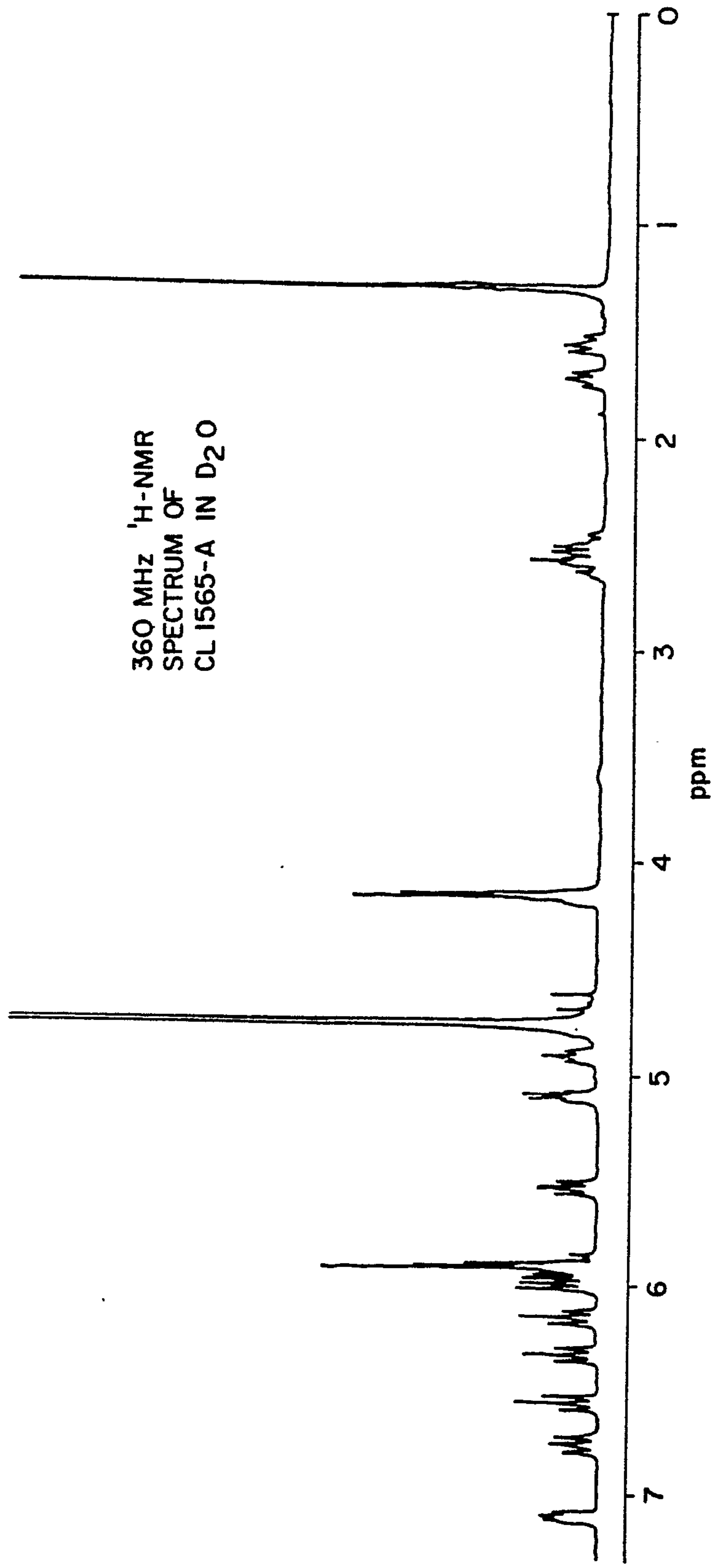


FIG. 3

$^{13}C$-NMR SPECTRUM OF
CL 1565-A (IN $D_2O$)

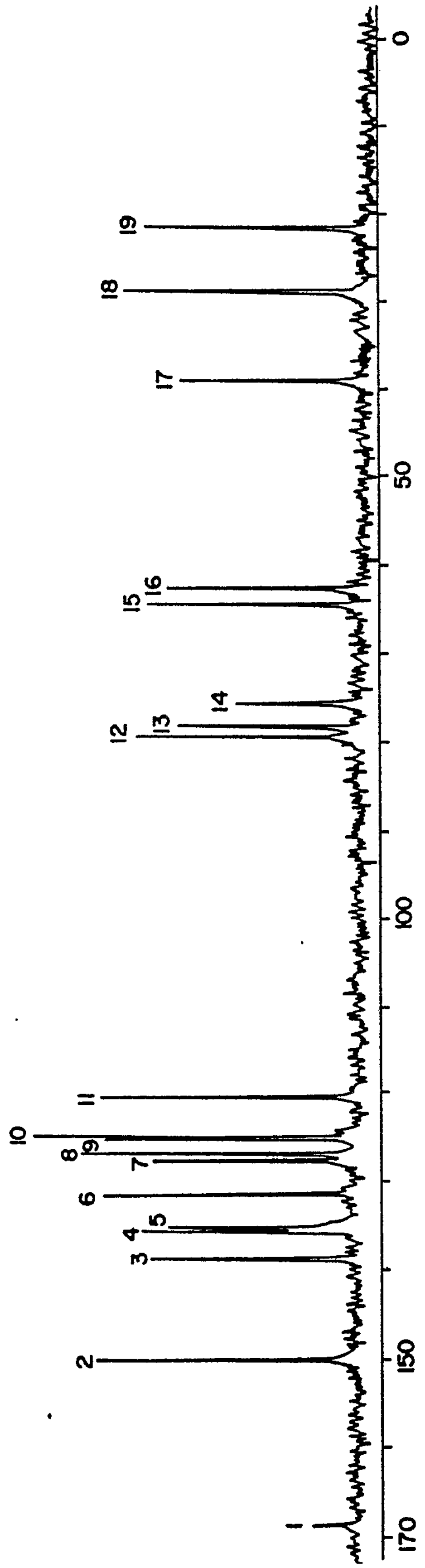


FIG. 4